# FASCICULE DE BREVET EUROPEEN

(11) **EP 2 181 725 B1**
(45) Date de publication et mention de la délivrance du brevet: **17.09.2014**
(21) Numéro de dépôt: 09172772.7
(22) Date de dépôt: 12.10.2009
(51) Int. Cl.: A61M 5/32

(54) **Aiguille d'injection**
Injektionsnadel
Injection needle

(30) Priorité: 28.10.2008 FR 0857306
(43) Date de publication de la demande: 05.05.2010
(73) Titulaire: Villette, Alain, 79700 Saint Pierre Les Echaubrognes (FR)
(72) Inventeur: Villette, Alain, 79700 Saint Pierre Les Echaubrognes (FR)
(74) Mandataire: Thinat, Michel

(56) Documents cités:
- EP-A- 1 736 192
- DE-A1-102005 027 147
- FR-A- 2 540 385

## Description

L'invention concerne une aiguille d'injection pour l'injection d'un produit pharmaceutique dans un tissu du corps humain ou animal.

Lors de certaines interventions chirurgicales et lors de soins dentaires, il est nécessaire, d'administrer au patient une anesthésie locale. Cette anesthésie se limite à la région visée et, dans le cas de soins dentaires, devrait se limiter à la dent ou aux dents à soigner. Le produit anesthésique est administré généralement par injection dans les tissus à anesthésier.

Lors de soins dermatologiques chez l'homme ou chez l'animal, il est parfois utile de disposer d'une aiguille conçue pour effectuer une injection de manière plus sûre et de manière moins douloureuse.

Et lors de soins dentaires, l'injection d'un produit anesthésique est habituellement faite dans les gencives afin de produire des effets dans les tissus mous périphériques. Il est alors utile de disposer d'une aiguille permettant d'atteindre le plus précisément possible le lieu d'anesthésie envisagé pour pouvoir limiter la quantité de produit anesthésique à administrer.

Toutefois, et indépendamment des désagréments que cela représente pour le patient, l'efficacité de l'anesthésie n'est pas toujours optimale, puisque le produit anesthésique se répand partiellement à distance de la dent à traiter au lieu d'y rester concentré. De plus, l'aiguille d'injection est souvent déviée, puisqu'elle se déforme en pénétrant dans les tissus, surtout dans les tissus mous.

En effet, les aiguilles selon la présente invention sont destinées principalement à l'administration de produits anesthésiques, mais par ailleurs aussi à d'autres applications, par exemple dermatologiques, où il s'agit de déposer un produit pharmaceutique à un endroit bien précis du corps humain ou animal.

Parmi les différentes techniques d'anesthésie, la présente invention intéresse plus particulièrement l'anesthésie intraligamentaire et l'anesthésie tronculaire pratiquées en chirurgie dentaire.

L'anesthésie intraligamentaire consiste à injecter un produit anesthésique dans le ligament enveloppant la dent à soigner. Le problème que pose l'anesthésie intraligamentaire avec des aiguilles classiques, est la difficulté que l'on a à faire pénétrer l'aiguille suffisamment profondément dans le ligament. Ceci occasionne, très souvent, même avec les aiguilles les plus fines, des fuites d'anesthésique. Pour remédier à ce problème, le praticien cherche à enfoncer plus profondément son aiguille et la plupart du temps elle se tord. Ceci impose un changement d'aiguille et une nouvelle puncture.

L'anesthésie tronculaire est une technique d'anesthésie du nerf dentaire inférieur avant son entrée dans le canal dentaire. Lors de la pénétration d'une aiguille dans un tissu mou, particulièrement lors de la réalisation de tronculaires ou anesthésies loco-régionales, l'aiguille est enfoncée assez profondément. Lors de sa pénétration, l'aiguille classique se déforme et suit une trajectoire courbe qui fait que l'injection n'est jamais réalisée exactement à l'endroit envisagé. Ceci est un facteur d'échec non négligeable. Pour y remédier, plusieurs solutions ont été proposées. Il faut savoir que la plupart des aiguilles dentaires utilisées sont d'un diamètre de 0,4 ou 0,5 mm. D'abord, augmenter le diamètre de l'aiguille pour augmenter sa rigidité. Il est recommandé d'utiliser préférentiellement le diamètre 0,51 mm. Classiquement le diamètre intérieur de cette aiguille est de 0,25 mm. Il est préconisé d'utiliser la technique « birotationnelle » BRIT (bi-rotational insertion technique, c'est-à-dire technique d'introduction bi-rotationnelle) qui consiste à faire tourner l'aiguille, lors de la pénétration, alternativement d'un demi-tour dans un sens et dans l'autre ceci pour que les effets de la déviation s'annulent. Cette manipulation est assez difficile à réaliser et seules les seringues à injection automatique la permettent.

On connaît encore par les documents EP 1 736 192 et FR 2 540 385 des aiguilles d'injection qui sont pourvues de moyens d'entraînement en rotation et présentent donc une structure complexe.

Le but de l'invention est de proposer une aiguille d'injection pour administrer des produits pharmaceutiques, qui remédie aux inconvénients décrits ci avant.

Le but de l'invention est atteint avec une aiguille d'injection pour l'injection d'un produit pharmaceutique dans un corps humain ou animal, l'aiguille comprenant un corps tubulaire ayant deux extrémités opposées dont une première extrémité destinée à être reliée à des moyens d'amenée de produit et une seconde extrémité destinée à pénétrer dans le corps humain ou animal, la seconde extrémité étant pourvue d'un biseau principal et d'un biseau secondaire formant une pointe de l'aiguille.

Conformément à l'invention, le biseau secondaire est disposé opposé au biseau principal et forme avec le biseau principal un bord incisant unique oblique par rapport à un axe longitudinal du corps tubulaire, le bord incisant s'étendant de part et d'autre d'un plan médian du biseau principal passant par l'axe longitudinal du corps tubulaire.

Grâce à la disposition opposée du biseau secondaire par rapport au biseau principal et l'étendue du bord incisant de part et d'autre d'un plan médian du biseau principal passant par l'axe longitudinal du corps tubulaire, l'extrémité avant de l'aiguille, vu dans le sens de la pénétration de l'aiguille dans un corps, est formée de façon à soumettre l'aiguille à deux forces approximativement opposées qui se réduisent réciproquement. De ce fait, la déviation de l'aiguille est pour le moins diminuée.

Cet effet positif de l'aiguille d'injection selon l'invention peut encore être amélioré en donnant à l'aiguille l'une au moins des caractéristiques ci-après, considérées isolément ou selon toute combinaison techniquement possible :
- le biseau secondaire forme avec l'axe longitudinal du corps tubulaire un angle inférieur à 10 degrés ;
- le biseau principal forme avec l'axe longitudinal du corps tubulaire un angle compris entre 5 degrés et 25 degrés ;
- le biseau principal forme avec l'axe longitudinal du corps tubulaire un angle compris entre 5 degrés et 10 degrés ;
- le biseau principal et le biseau secondaire enferment entre eux un angle compris entre 15 degrés et 30 degrés ;
- le corps tubulaire présente un diamètre intérieur inférieur aux valeurs normalisées pour les différents calibres d'aiguille ;
- l'aiguille est pourvue d'un marquage de positionnement disposé, par rapport à l'axe longitudinal de l'aiguille, opposé au biseau principal.

D'autres caractéristiques et avantages de la présente invention ressortiront de la description ci-après de deux aiguilles constitutives d'exemples de réalisation. La description est faite en référence aux dessins dans lesquels :
la figure 1 représente une première aiguille d'injection selon l'invention en une vue latérale avec une vue sur le biseau secondaire,
la figure 2 représente la première aiguille en une vue latérale avec vue sur le biseau principal,
la figure 3 représente la première aiguille avec une vue de dessus sur le biseau principal,
la figure 4 représente la première aiguille en une vue axiale sur les deux biseaux,
la figure 5 représente une seconde aiguille d'injection selon l'invention en une vue latérale avec une vue sur le biseau secondaire,
la figure 6 représente la seconde aiguille en une vue latérale avec vue sur le biseau principal,
la figure 7 représente la seconde aiguille avec une vue de dessus sur le biseau principal,
la figure 8 représente la seconde aiguille en une vue axiale sur les deux biseaux, et
la figure 9 représente une aiguille d'injection avec un marquage de positionnement.

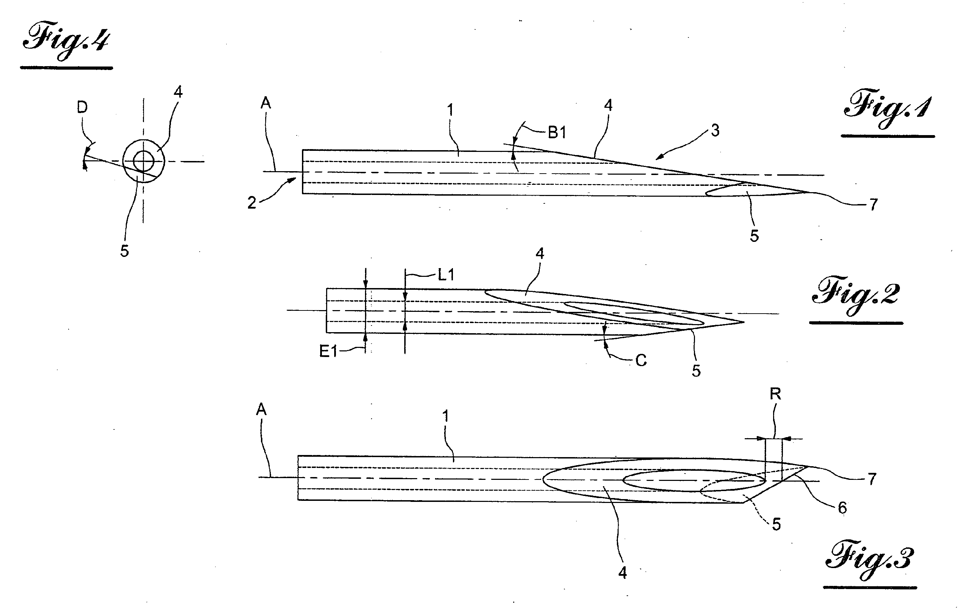

Les figures 1 à 4 représentent une aiguille d'injection pour une anesthésie intra-ligamentaire. L'aiguille comprend un corps tubulaire 1 avec un axe longitudinal A et deux extrémités opposées 2, 3 dont une première extrémité 2 est conformée pour être reliée à des moyens d'amenée de produit, par exemple à une seringue ou à un conduit flexible qui, lui, est relié à une bouteille ou tout autre type de contenant ou à un appareil automatique conformé pour administrer un produit pharmaceutique de manière programmable.

La seconde extrémité 3, qui est destinée à pénétrer dans un corps humain ou animal, est pourvue d'un biseau principal 4 et d'un biseau secondaire 5 formant à la fois un bord incisant unique 6 et une pointe 7 de l'aiguille.

Le corps tubulaire 1 de l'aiguille d'injection est un corps essentiellement cylindrique creux et de révolution autour de l'axe longitudinal A. Le corps tubulaire 1 présente ainsi un diamètre extérieur E1, et un diamètre intérieur L1. Alors que le diamètre extérieur E1 indique le calibre de l'aiguille d'injection, le diamètre intérieur L1 indique la taille de la lumière du corps tubulaire 1 par laquelle le produit pharmaceutique doit pouvoir passer lors d'une injection.

Les aiguilles d'injection utilisées en chirurgie dentaire pour une anesthésie intra-ligamentaire sont réalisées à partir de tubes ayant un diamètre extérieur de 0,3 mm. Conformément à l'invention, un tel tube est pourvu, à l'extrémité destinée à pénétrer dans un corps humain ou animal, d'un biseau principal 4 et d'un dispositif secondaire 5 disposés opposés l'un par rapport à l'autre et avec un angle D relativement faible entre les deux biseaux. De cette manière, l'intersection entre les deux biseaux 4, 5 résulte en un bord incisant 6 oblique par rapport à l'axe longitudinal A du corps tubulaire 1 de l'aiguille. L'orientation du bord incisant 6 dépendant essentiellement de l'orientation relative des deux biseaux l'un par rapport à l'autre, l'orientation par rapport à l'axe A du corps tubulaire 1 s'éloigne de plus en plus d'une disposition perpendiculaire par rapport à l'axe A au fur et à mesure que l'angle D entre les biseaux augmente.

Cependant, l'orientation du bord incisant 6 dépend également de l'angle que chacun des deux biseaux forme avec l'axe longitudinal A du corps tubulaire 1. Dans le cas des aiguilles d'injection pour anesthésie intra-ligamentaire, l'angle B entre le biseau principal 4 et l'axe longitudinal A et l'angle C entre le biseau secondaire 5 et l'axe longitudinal A sont au moins approximativement égaux et les plus faibles possibles, tenant compte de contraintes techniques de réalisation. En donnant aux deux angles B et C des valeurs inférieures à 10° par rapport à l'axe longitudinal A, on réalise des aiguilles très plates que l'on peut insérer profondément dans un ligament.

En même temps, les faibles angles entre chacun des deux biseaux 4, 5 et l'axe longitudinal A, et plus particulièrement le faible angle C entre le biseau secondaire 5 et l'axe longitudinal A, ne sont possibles que par l'utilisation d'un tube spécial ayant une paroi plus épaisse que ce n'est le cas des tubes selon la norme ISO 9626 qui fixe par exemple les valeurs suivantes :

| Dimension théorique (mm) | Calibre | Diamètre extérieur (mm) | | Diamètre intérieur (mm) | Epaisseur de la paroi (mm) | |
|---|---|---|---|---|---|---|
| | | min | max | | min | max |
| 0,3 | 30 | 0,298 | 0,320 | 0,133 | 0,083 | 0,094 |
| 0,5 | 25 | 0,500 | 0,530 | 0,232 | 0,134 | 0,149 |

En utilisant, pour la réalisation des aiguilles selon l'invention, un tube ayant une paroi plus épaisse car ayant un diamètre intérieur L1 réduit par rapport à celui des aiguilles normalisées, c'est-à-dire en diminuant le diamètre de la lumière de l'aiguille, on peut rapprocher le biseau secondaire 5 de l'axe longitudinal A du corps tubulaire 1, ce qui fait diminuer la longueur de la lumière à l'extrémité 3 de l'aiguille.

De cette manière, on peut obtenir des aiguilles d'injection ayant par exemple les caractéristiques suivantes :

| Dimension théorique (mm) | Calibre | Diamètre extérieur (mm) | | Diamètre intérieur (mm) | Epaisseur de la paroi (mm) | |
|---|---|---|---|---|---|---|
| | | min | max | | min | max |
| 0,3 | 30 | 0,298 | 0,320 | 0,110 | 0,094 | 0,110 |
| 0,5 | 25 | 0,500 | 0,530 | 0,200 | 0,150 | 0,165 |

De plus, on peut réduire l'angle C entre le biseau secondaire 5 et l'axe longitudinal A par rapport aux aiguilles classiques. Une réflexion analogue s'applique à l'orientation du biseau principal 4 et l'angle B1 qu'il forme avec l'axe longitudinal A. Dans l'exemple de réalisation représenté sur les figures 1 à 4, les deux angles B1 et C sont égaux avec la même tolérance et ont des valeurs numériques de 7° ± 1°.

L'aiguille d'injection selon l'invention, conformée pour pouvoir être utilisée en anesthésie intra-ligamentaire, bénéficie principalement de deux avantages. Tout d'abord, la réalisation de l'aiguille avec une extrémité en forme de lame permet de la faire pénétrer plus profondément dans un tissu, donc de supprimer les fuites lors de l'injection, et de l'exposer ensuite le moins possible à des composantes transversales des efforts de résistance que l'aiguille rencontre en pénétrant de plus en plus profondément dans le tissu. Par ailleurs, l'utilisation d'une aiguille à paroi plus épaisse fait bénéficier la personne ou l'animal à traiter des avantages d'un tube plus rigide et est donc moins objet à une déformation, notamment flexion, source principale de la déviation des aiguilles classiques.

En résumé, les aiguilles selon l'invention sont améliorées par rapport aux aiguilles classiques sous deux aspects différents dont chacun contribue indépendamment de l'autre à une plus grande linéarité du trajet de pénétration. En même temps, ces deux avantages s'additionnent et s'amplifient.

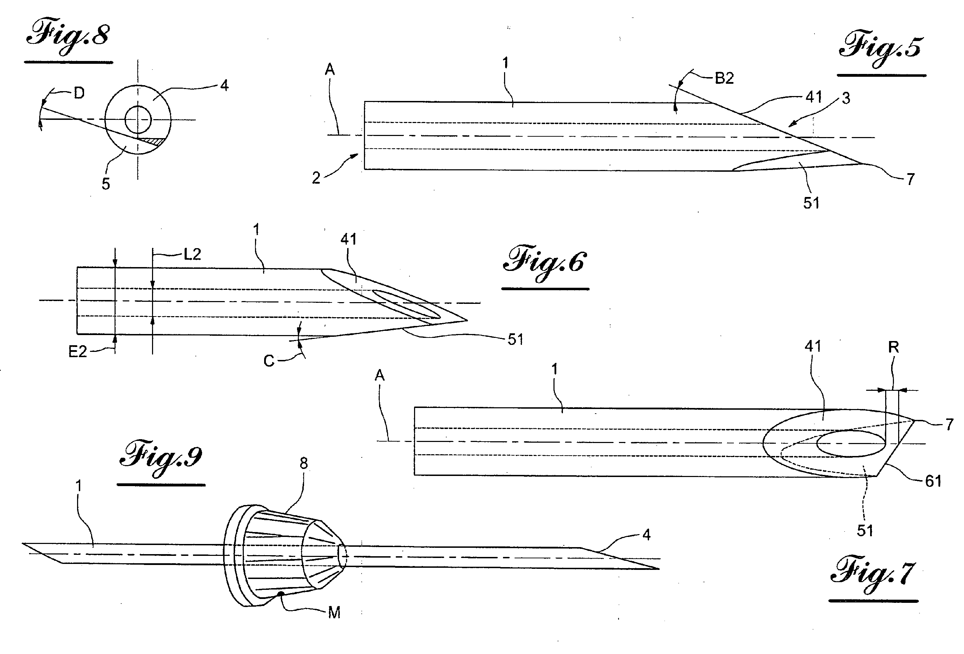

Pour profiter pleinement des avantages apportés par la présente invention, les aiguilles selon l'invention sont avantageusement pourvues, sur une embase 8 de l'aiguille, dans laquelle le corps tubulaire est logé, d'un marquage de positionnement M visible sur la figure 9. Le marquage se situe sur le dos de l'aiguille par rapport au biseau principal 4. Ceci permet au praticien de savoir que le biseau principal 4 de l'aiguille est bien parallèle à la muqueuse.

Lorsque l'aiguille d'injection selon l'invention est réalisée pour des applications en anesthésie tronculaire, c'est-à-dire pour déposer un produit pharmaceutique et notamment anesthésique près d'un tronc nerveux, on utilise classiquement des aiguilles de calibre 25, c'est-à-dire ayant un diamètre extérieur de 0,5 mm. Cependant, pour faire bénéficier ces aiguilles des dispositions de l'invention, on les réalise à partir de tubes ayant un diamètre intérieur de l'ordre de 0,20 mm seulement Il en résultent des aiguilles à parois plus épaisses que les parois des aiguilles normalisées. Cette augmentation de l'épaisseur de la paroi de l'aiguille permet d'abord d'augmenter sa rigidité et de diminuer ainsi sa flexion lors d'une pénétration dans un tissu. Ensuite, cette augmentation de l'épaisseur de la paroi permet de rapprocher le biseau secondaire de l'axe longitudinal A du corps tubulaire 1, sans pénétrer la lumière.

Ainsi, on retrouve pour l'aiguille d'injection réalisée selon le second exemple de réalisation représenté sur les figues 5 à 8 les mêmes dispositions de principe que celles décrites par rapport au premier exemple de réalisation.

Toutefois, en raison d'un calibre E2 plus grand de l'aiguille selon le second exemple de réalisation par rapport au calibre E1 de l'aiguille du premier exemple de réalisation et en raison d'un diamètre intérieur L2 proportionnellement moins grand que le diamètre intérieur L1 des aiguilles du premier exemple de réalisation, l'angle du biseau principal 41 de l'aiguille selon le second exemple de réalisation ne peut pas être aussi faible que l'angle du biseau principal selon le premier exemple de réalisation. Ainsi, l'angle B2 que le biseau principal 41 forme avec l'axe longitudinal A, est de l'ordre de 20° à 25°, par exemple 21° ± 1°.

Contrairement à cela, l'angle C que le biseau secondaire 51 forme avec l'axe longitudinal A du conduit 1 est égal à celui des aiguilles du premier exemple de réalisation, à savoir inférieur à 10°, de préférence 7° + 1°. Ainsi, aussi bien pour le biseau secondaire 5 selon le premier exemple de réalisation que pour le biseau secondaire 51 selon le second exemple de réalisation, un angle C formé avec l'axe longitudinal A allant jusqu'à 6° est envisageable.

Bien que le biseau principal et le biseau secondaire de l'aiguille selon le second exemple de réalisation n'aient pas des angles aussi faibles que selon le premier exemple de réalisation, l'extrémité de l'aiguille est néanmoins soumise à des forces opposées presque équivalentes et notamment avec de faibles composantes transversales, ce qui diminue de manière significative le risque de déviation de l'aiguille lors d'une pénétration dans des tissus d'un corps humain ou animal.

## Revendications

1. Aiguille d'injection pour l'injection d'un produit pharmaceutique dans un corps humain ou animal, l'aiguille comprenant un corps tubulaire (1) ayant deux extrémités opposées (2, 3) dont une première extrémité (2) destinée à être reliée à des moyens d'amenée de produit et une seconde extrémité (3) destinée à pénétrer dans le corps humain ou animal, la seconde extrémité (3) étant pourvue d'un biseau principal (4) et d'un biseau secondaire (5) disposé opposé au biseau principal (4) et formant avec le biseau principal (4) une pointe (7) de l'aiguille et un bord incisant unique (6) oblique par rapport à un axe longitudinal (A) du corps tubulaire,
**caractérisée en ce que** le bord incisant (6) s'étend de part et d'autre d'un plan médian du biseau principal (4) passant par l'axe longitudinal (A) du corps tubulaire (1).

2. Aiguille selon la revendication 1, **caractérisée en ce que** le biseau secondaire (5) forme avec l'axe longitudinal (A) du corps tubulaire (1) un angle (C) inférieur à 10 degrés.

3. Aiguille selon la revendication 2, **caractérisée en ce que** le biseau principal (4) forme avec l'axe longitudinal (A) du corps tubulaire (1) un angle (B) compris entre 5 degrés et 25 degrés.

4. Aiguille selon la revendication 2, **caractérisée en ce que** le biseau principal (4) forme avec l'axe longitudinal (A) du corps tubulaire (1) un angle (B1) compris entre 5 degrés et 10 degrés.

5. Aiguille selon l'une quelconque des revendications 1 à 4, **caractérisée en ce que** le biseau principal (4) et le biseau secondaire (5) enferment entre eux un angle (D) compris entre 15 degrés et 35 degrés.

6. Aiguille selon l'une quelconque des revendications 1 à 5, **caractérisée en ce qu'**elle est pourvue d'un marquage de positionnement (M) disposé, par rapport à un axe longitudinal (A) de l'aiguille, opposé au biseau principal (4).

## Patentansprüche

1. Injektionsnadel für die Injektion eines pharmazeutischen Produkts in einen Körper eines Menschen oder eines Tiers, wobei die Nadel einen rohrförmigen Körper (1) mit zwei gegenüberliegenden Enden (2, 3) umfasst, von denen ein erstes Ende (2) zur Verbindung mit Produktzufuhrmitteln bestimmt ist und ein zweites Ende (3) zum Eindringen in den Körper des Menschen oder des Tiers bestimmt ist, wobei das zweite Ende (3) mit einer Hauptschrägkante (4) und einer sekundären Schrägkante (5) ausgestattet ist, die gegenüber der Hauptschrägkante (4) angeordnet ist und mit der Hauptschrägkante (4) eine Spitze (7) der Nadel und einen im Verhältnis zu einer Längsachse (A) des rohrförmigen Körpers einzigen schrägen Schnittrand (6) bildet,
**dadurch gekennzeichnet, dass** sich der Schnittrand (6) auf der einen und der anderen Seite einer mittleren Ebene der Hauptschrägkante (4) durch die Längsachse (A) des rohrförmigen Körpers (1) erstreckt.

2. Nadel nach Anspruch 1, **dadurch gekennzeichnet, dass** die sekundäre Schrägkante (5) mit der Längsachse (A) des rohrförmigen Körpers (1) einen Winkel (C) kleiner als 10 Grad bildet.

3. Nadel nach Anspruch 2, **dadurch gekennzeichnet, dass** die Hauptschrägkante (4) mit der Längsachse (A) des rohrförmigen Körpers (1) einen Winkel (B) zwischen 5 Grad und 25 Grad inklusive bildet.

4. Nadel nach Anspruch 2, **dadurch gekennzeichnet, dass** die Hauptschrägkante (4) mit der Längsachse (A) des rohrförmigen Körpers (1) einen Winkel (B1) zwischen 5 Grad und 10 Grad inklusive bildet.

5. Nadel nach einem der Ansprüche 1 bis 4, **dadurch gekennzeichnet, dass** die Hauptschrägkante (4) und die sekundäre Schrägkante (5) zwischen sich einen Winkel (D) zwischen 15 Grad und 35 Grad bilden.

6. Nadel nach einem der Ansprüche 1 bis 5, **dadurch gekennzeichnet, dass** sie mit einer Positionierungsmarkierung (M) ausgestattet ist, die im Verhältnis zu einer Längsachse (A) der Nadel der Hauptschrägkante (4) gegenüberliegend angeordnet ist.

## Claims

1. An injection needle for injecting a pharmaceutical product into a human or animal body, the needle comprising a tubular body (1) having two opposite ends (2, 3), including a first end (2) designed to be connected to product intake means and a second end (3) designed to penetrate the human or animal body, the second end (3) being provided with a main bevel (4) and a secondary bevel (5) positioned opposite the main bevel (4) and forming, with the main bevel (4), a tip (7) of the needle and a single incision edge (6) that is oblique relative to a longitudinal axis (A) of the tubular body,
**characterized in that** the incision edge (6) extends on either side of a median plane of the main bevel (4) passing through the longitudinal axis (A) of the tubular body (1).

2. The needle according to claim 1, **characterized in that** the secondary bevel (5) forms an angle (C) smaller than 10 degrees with the longitudinal axis (A) of the tubular body (1).

3. The needle according to claim 2, **characterized in that** the main bevel (4) forms an angle (B) comprised between 5 degrees and 25 degrees with the longitudinal axis (A) of the tubular body (1).

4. The needle according to claim 2, **characterized in that** the main bevel (4) forms an angle (B1) comprised between 5 degrees and 10 degrees with the longitudinal axis (A) of the tubular body (1).

5. The needle according to any one of claims 1 to 4, **characterized in that** the main bevel (4) and the secondary bevel (5) contain an angle (D) comprised between 15 degrees and 35 degrees between them.

6. The needle according to any one of claims 1 to 5, **characterized in that** it is provided with a positioning mark (M) positioned opposite the main bevel (4) relative to a longitudinal axis (A) of the needle.
